# EUROPEAN PATENT SPECIFICATION

(11) **EP 2 890 354 B1**
(45) Date of publication and mention of the grant of the patent: **20.07.2016**
(21) Application number: 13763147.9
(22) Date of filing: 30.08.2013
(51) Int. Cl.: A61J 7/00, A61K 49/00, B65D 85/816, G01N 24/00, G01F 19/00

(54) **CONTAINER WITH CONCENTRATED SUBSTANCE AND METHOD OF USING THE SAME**
BEHÄLTER MIT EINEM KONZENTRIERTEN STOFF UND VERFAHREN ZUR VERWENDUNG DAVON
RÉCIPIENT AYANT UNE SUBSTANCE CONCENTRÉE ET SON PROCÉDÉ D'UTILISATION

(30) Priority: 30.08.2012 US 201213599045
(43) Date of publication of application: 08.07.2015
(73) Proprietor: OTSUKA PHARMACEUTICAL CO., LTD., Tokyo 101-8535 (JP)
(72) Inventor: HOLLANDER, Scott Wayne, Princeton, New Jersey 08540 (US); TIMBERLAKE, Joel Robert, West Windsor, New Jersey 08550 (US); KADASH, Marjory, Skillman, New Jersey 08558 (US)
(74) Representative: Hoffmann Eitle
(86) International application number: PCT/US2013/057529
(87) International publication number: WO 2014/036402

(56) References cited:
- EP-A2- 0 255 493
- WO-A1-2011/122639
- WO-A2-2008/002493
- GB-A- 2 435 027
- US-A1- 2005 284 302

## Description

### CROSS-REFERENCE TO RELATED APPLICATION

This application claims the benefit of priority to, and is a continuation-in-part (CIP) application of, U.S. Utility Application No. 13/599,045, filed on August 30, 2012, entitled "CONTAINER WITH CONCENTRATED SUBSTANCE AND METHOD OF USING THE SAME".

### BACKGROUND

Some embodiments described herein relate to a container with a unit dose of a concentrated substance disposed therein that can receive a volume of liquid to dilute the concentrated substance to a desired concentration for oral consumption by a user.

Some known concentrated substances, such as, for example, concentrated medicaments and/or oral contrast agents, are provided to healthcare facilities in bulk containers that can hold a large quantity of the medicament or contrast agent. For example, some radiological contrast agents are typically provided in such a manner, such as those containing barium (e.g. barium sulphate) or iodine. Typically, such concentrated materials require the use of a separate container for dilution and/or consumption of the medicament or contrast agent. Such use of multiple containers for preparing a concentrated medicament or contrast agent for oral consumption can present various undesirable results, such as, for example, improper dilution strength, separation of the medicament or contrast agent from identifying labels and/or separation of the medicament or contrast agent from instructions for use.

Some known iodine based contrast media are provided as solutions that require dilution, and sometimes are provided with measuring cups to facilitate accurate dilution to different strengths. Some known ionic iodinated contrast media are available that contain flavoring, but are not provided in packaging that is ready for patient consumption. There are also some known containers that include a diluting solution in which an oral contrast agent can be mixed, but such containers still require that the active contrast agent be drawn from a large volume package. Such containers may also require drawing oral drug doses from a bottle intended and labeled for intravenous injection rather than for oral consumption. Such a situation can be undesirable, for example, if the doses are prepared in areas of a medical facility outside of the radiology suite and partially filled containers of contrast agent are kept at hand. In addition, by providing the diluting solution in such a bottle, rather than the contrast agent, such a container does not permit the range of beverage choices (flavors, carbonation, sweetness) that may lead to higher patient compliance (e.g., full consumption of the prescribed dose and concentration).

Document EP0255493 discloses a container for the simultaneous administration of barium sulphate suspension and air in the double contrast x-ray examination of the upper digestive tract.

Accordingly, a need exists for a container that can include a unit dose of a concentrated substance, such as, a contrast agent, and can be used for transport, dilution and oral consumption of the concentrated substance.

### SUMMARY

The invention is defined by an apparatus according to claim 1 and a method according to claim 13. Where in the following the word invention is used and/or features are presented as optional this should be interpreted in such way that protection is sought for the invention as claimed.
Apparatus and methods are described herein for a container with a unit dose of a concentrated substance that can be diluted and orally consumed using the container. The apparatus includes a container body that defines an opening in fluid communication with an interior of the container body. A cap is coupled to the container body and encloses the opening. A unit dose of a concentrated contrast agent is disposed within the interior of the container body. The unit dose of concentrated contrast agent can be diluted to a select dilution strength with a volume of a liquid receivable through the opening and within the interior of the container body. The apparatus includes a barrier in which the container body and cap are disposed to protect the contrast agent from light and/or moisture.

In an example, an apparatus includes a container body that defines an opening in fluid communication with an interior of the container body. A cap is coupled to the container body and encloses the opening. A unit dose of a dilutable substance is disposed within the interior of the container body. The unit dose of the dilutable substance is configured to be diluted to a select dilution strength with a volume of a liquid that is receivable through the opening and within the interior of the container body. A barrier member defines an interior, and the container body and the cap are collectively disposed within the interior of the barrier member.

In an example, an apparatus consists essentially of a container body that defines an opening in fluid communication with an interior of the container body; a cap is coupled to the container body and encloses the opening; a unit dose of a dilutable substance is disposed within the interior of the container body, the unit dose of the dilutable substance is configured to be diluted to a select dilution strength with a volume of a liquid that is receivable through the opening and within the interior of the container body; a barrier member defines an interior, the container body and the cap are collectively disposed within the interior of the barrier member; and instructions for use are disposed within the interior of the barrier member; wherein the container body is configured to be used by a patient to orally consume the unit dose of the dilutable substance through the opening of the container body when the unit dose of the dilutable substance has been diluted with the volume of liquid, and the barrier member is configured to shield the unit dose of the dilutable substance from at least one of light or moisture.

In an example, an apparatus consists of a container body that defines an opening in fluid communication with an interior of the container body; a cap is coupled to the container body and encloses the opening; a unit dose of a dilutable substance is disposed within the interior of the container body, the unit dose of the dilutable substance is configured to be diluted to a select dilution strength with a volume of a liquid that is receivable through the opening and within the interior of the container body; a barrier member defines an interior, the container body and the cap are collectively disposed within the interior of the barrier member; and instructions for use are disposed within the interior of the barrier member; wherein the container body is configured to be used by a patient to orally consume the unit dose of the dilutable substance through the opening of the container body when the unit dose of the dilutable substance has been diluted with the volume of liquid, and the barrier member is configured to shield the unit dose of the dilutable substance from at least one of light or moisture.

In an example, a method includes removing a cap from a container body. The container body has a unit dose of a concentrated medicament or a concentrated contrast agent disposed within an interior defined by the container body. A volume of a liquid is added to the interior of the container body such that the concentrated medicament or the concentrated contrast agent is diluted to a select dilution strength. The container body is provided to a patient to orally consume the diluted medicament or concentrated contrast agent.

In an example, a method includes removing a cap from a container body. The container body has a unit dose of a functional excipient free concentrated contrast agent disposed within an interior defined by the container body. A volume of a liquid is added to the interior of the container body such that the functional excipient free concentrated contrast agent is diluted to a select dilution strength. The container body is provided to a patient to orally consume the diluted functional excipient free concentrated contrast agent.

In an example a method includes removing a cap from a container body. The container body has a unit dose of a dilutable powder contrast agent disposed within an interior defined by the container body. A volume of a liquid is added to the interior of the container body such that the unit dose of the powder contrast agent is diluted to a select dilution strength with a volume of liquid that is receivable through the opening and substantially fills the interior of the container body. The container body is provided to a patient to orally consume the diluted powder contrast agent.

### BRIEF DESCRIPTION OF THE DRAWINGS

FIG. 1 is a schematic illustration of a container with a concentrated substance, according to an embodiment.
FIG. 2 is a front perspective view of a container with a concentrated substance, according to an embodiment, shown in a first configuration.
FIG. 3 is a partially exploded side view of the container of FIG. 2, shown in a second configuration with a liquid added to the container.
FIG. 4 is a side view of the container of FIG. 2, shown in a third configuration with the concentrated substance diluted.
FIG. 5 is a side view of a container with a concentrated substance, according to another embodiment.
FIG. 6 is a front perspective view of a container with a concentrated substance, according to another embodiment.
FIG. 7 is a flow chart illustrating a method for using a container with a concentrated substance, according to an embodiment.

### DETAILED DESCRIPTION

Apparatus and methods are described herein for a container for transport and oral consumption of a unit dose of a concentrated substance, such as for example, contrast agent. The apparatus includes a container having a container body that defines an interior volume and an opening in fluid communication with the interior volume. A cap is removably coupled to the container body to close or cover the opening. The interior volume contains a unit dose of a concentrated contrast agent. The concentrated substance can be diluted to a selected concentration or dilution strength with a volume of liquid received in the interior volume through the opening of the container body. The interior volume, including the concentrated substance, has sufficient head space to receive the volume of liquid to dilute the concentrated substance to the selected dilution strength. The apparatus includes a barrier member that can shield the contents of the container the concentrated contrast agent) from exposure to moisture and/or light. In some embodiments, the container body itself can act as a barrier. In an example not part of the invention, the container body can be formed with a material that can shield the concentrated substance from moisture and/or light.

In an example an apparatus includes a container having a container body that defines an opening in fluid communication with an interior of the container body. A cap can be removably coupled to the container body and enclose the opening. The apparatus can also include a unit dose of a concentrated medicament or a concentrated contrast agent disposed within the interior of the container body. The unit dose of the concentrated medicament or the concentrated contrast agent can be diluted to a select dilution strength with a volume of a liquid (generally, a diluent) that is receivable through the opening and within the interior of the container body.

In some embodiments, the unit dose and the interior of the container body are sterile. Similarly stated, the unit dose and the interior of the container body are considered to be sterile prior to the removal of the cap for the first time. In other embodiments, the unit dose and the interior of the container body can be non-sterile. In some embodiments, the diluent can be sterile, while in other embodiments, the diluent can be non-sterile.

the unit dose is a concentrated contrast agent. In some embodiments, the concentrated contrast agent is a concentrated radiocontrast agent, a concentrated magnetic resonance imaging (MRI) agent, or a concentrated ultrasound imaging agent. In some embodiments, the concentrated radiocontrast agent is an agent based on iodine or barium (e.g. barium sulphate). In some embodiments, the iodine-based radiocontrast agent is ionic or non-ionic. In some embodiments, the ionic iodine-based radiocontrast agent is selected from the group consisting of amidotrizoate (i.e., the salt form of diatrizoic acid), metrizoate, ioxaglate (i.e., the salt form of ioxaglic acid), and ioxithalamate. In some embodiments, the ionic iodine-based radiocontrast agent is in a free-base form (e.g., an acid form) or a salt form (e.g. sodium salt form, meglumine salt form, and/or other salt forms). In some embodiments, the non-ionic iodine-based radiocontrast agent is selected from the group consisting of iopamidol, iohexol, ioxilan, iopromide, iotrolan, iopentol, ioversol, iomeprol, iobitridol, and iodixanol. In one embodiment, the concentrated contrast agent is an agent with lower osmolality, such as the non-ionic agents, which tend to have fewer side-effects. In some embodiments, the ultrasound imaging agent is selected from the group consisting of sulful hexafluoride (e.g. in the form of microbubbles), simethicone-coated cellulose, polydextrose (e.g. as a suspension of polydestrose in purified water), polyethylene glycol (e.g. as an iso-molar solution), perflutren (also known as OPTISON), perflexane (also known as IMAGENT), and perflutren (also known as DEFINITY).

In some embodiments, the concentrated radiocontrast agent is a concentrated MRI agent based on metal. In some embodiments, the concentrated MRI agent comprises a metal selected from manganese, iron, gadolinium, platinum, dysprosium, and holmium. Those metals can be in various oxidation states and/or various forms, such as free-base forms (e.g., an acid form), salts (e.g., sodium, meglumine, or acetate salts), hydroxides, oxides, or chelated derivatives (complex with coordinated ligands). For example, gadolinium and dysprosium can be Gd (III) and Dy (III), respectively, or any chelated derivatives. In some embodiments, the concentrated MRI agent is selected from the group consisting of gadoterate, gadodiamide, gadobenate, gadopentetate, gadoteridol, gadofosveset, gadoversetamide, gadoxetate, gadobutrol, gadopentetic acid, gadobenic acid, gadoteric acid, gadoxetic acid, iron oxide, ion platinum, manganese chloride (MnCl₂), mangafodipir trisodium (Mn-DPDP), gadolinium diethylenetriaminepentacetate (Gd-DTPA), Gd-DTPA bismethylamide (Gd-DTPA-BMA), Gd-DTPA 2-(R)-[(4,4-diphenylcyclohexyl)phosphonooxymethyl] (Gd-DTPA-PcHexPh₂), gadolinium 1,4,7,10-tetraazacyclododecane-1,4,7,10-tetrakis (ethylacetamidoacetate) (Gd-DOTA), dysprosium diethylenetriaminepentacetate 2-(R)-[(4,4-diphenylcyclohexyl)phosphonooxymethyl] (Dy-DTPA-PcHexPh₂), dysprosium 2-(R)-[(4,4-diphenylcyclohexyl)phosphonooxymethyl] diethylenetriamine hexaacetate (Dy-TTHA-PcHexPh₂), dysprosium oxide, dysprosium hydroxide, Ho-acetylacetonate in polylactate, ferumoxsil, ferristene, and ferric ammonium citrate (FAC).

In some embodiments, the concentrated contrast agent comprises a contrast agent based on fluorine, bromine, iodine, barium, magnesium, manganese, iron, gadolinium, dysprosium, holmium, or combinations thereof. In some embodiments, the concentrated contrast agent is selected from the group consisting of iopamidol, iohexol, ioxilan, iopromide, iotrolan, iodixanol, iopentol, ioversol, iomeprol, iobitridol, amidotrizoate, metrizoate, ioxaglate, ioxithalamate, ioxaglic acid, barium sulphate (BaSO₄), FAC, MnCl₂, Gd-DTPA, ferumoxsil, ferristene, perfluoro-octylbromide, gadoterate, gadodiamide, gadobenate, gadopentetate, gadoteridol, gadofosveset, gadoversetamide, gadoxetate, gadobutrol, gadopentetic acid, gadobenic acid, gadoteric acid, gadoxetic acid, iron oxide, ion platinum, Mn-DPDP, Gd-DTPA-BMA, Gd-DTPA-PcHexPh₂, Gd-DOTA, Dy-DTPA-PcHexPh₂, Dy-TTHA-PcHexPh₂, dysprosium oxide, dysprosium hydroxide, Ho-acetylacetonate in polylactate, sulful hexafluoride (e.g. in the form of microbubbles), simethicone-coated cellulose, polydextrose (e.g. as a suspension of polydestrose in purified water), polyethylene glycol (e.g. as an iso-molar solution), perflutren (also known as OPTISON), perflexane (also known as IMAGENT), and perflutren (also known as DEFINITY). In some embodiments, the concentrated contrast agent is substantially free of functional excipients or additives. In some embodiments, the concentrated contrast agent is substantially free of functional excipients or additives.

In some embodiments, the concentrated contrast agent is in a powder form, in a liquid form, or in a tablet form. In some embodiments, the concentrated contrast agent is an iodinated contrast agent. In some embodiments, the concentrated contrast agent is in a powder form and is selected from the group consisting of selected from the group consisting of iohexol, iopamidol, ioxilan, iopromide, iotrolan, iodixanol, iopentol, ioversol, iomeprol, iobitridol, amidotrizoate, metrizoate, ioxaglate, ioxaglic acid, and ioxithalamate.

In some embodiments, the concentrated contrast agent is selected from the group consisting of iohexol, iopamidol, ioxilan, iopromide, iotrolan, iodixanol, iopentol, ioversol, iomeprol, iobitridol, amidotrizoate, metrizoate, ioxaglate, ioxaglic acid, and ioxithalamate.

In some embodiments, the concentrated contrast agent is iohexol, and the unit dose of iohexol is from about 0.5 gI to about 25 gI with the volume of the liquid receivable from about 80 mL to about 2,000 mL, is from about 0.7 gI to about 25 gI with the volume of the liquid receivable from about 100 mL to about 1,100 mL, is from about 0.9 gI to about 21 gI with the volume of the liquid receivable from about 100 mL to about 1,000 mL, is from about 4.5 gI to about 9 gI with the volume of the liquid receivable from about 120 mL to about 1,000 mL, and all value in between.

In some embodiments, the unit dose of iohexol is from about 0.2 gI to about 75 gI with the volume of the liquid receivable from about 4 mL to about 250 mL, is from about 0.3 gI to about 50 gI with the volume of the liquid receivable from about 4 mL to about 220 mL, is from about 0.7 gI to about 36 gI with the volume of the liquid receivable from about 4 mL to about 210 mL, and all values in between.

In some embodiments, the concentrated contrast agent is iopamidol, iopentol, iopromide, ioversol, ioxilan, iomeprol, iobitridol, iotrolan, and/or iodixanol, and the unit dose of iopamidol, iopentol, iopromide, ioversol, ioxilan, iomeprol, iobitridol, iotrolan, and/or iodixanol is independently selected from about 0.5 gI to about 40 gI with the volume of the liquid receivable from about 30 mL to about 1,500 mL, is from about 0.7 gI to about 35 gI with the volume of the liquid receivable from about 80 mL to about 800 mL, is from about 0.9 gI to about 31 gI with the volume of the liquid receivable from about 100 mL to about 600 mL, and all values in between.

In some embodiments, the concentrated contrast agent is iopamidol, iopentol, iopromide, ioversol, ioxilan, iomeprol, iobitridol, iotrolan, and/or iodixanol, and the unit dose of iopamidol iopentol, iopromide, ioversol, ioxilan, iomeprol, iobitridol, iotrolan, and/or iodixanol is independently selected from about 0.1 gI to about 70 gI with the volume of the liquid receivable from about 20 mL to about 300 mL, is from about 0.15 gI to about 65 gI with the volume of the liquid receivable from about 30 mL to about 250 mL, is from about 0.2 gI to about 60 gI with the volume of the liquid receivable from about 40 mL to about 200 mL, and all values in between.

In some embodiments, the concentrated contrast agent is amidotrizoate, ioxaglic acid, and/or ioxithalamate, and the unit dose of amidotrizoate, ioxaglic acid, and/or ioxithalamate is from about 0.2 gI to about 45 gI with the volume of the liquid receivable from about 50 mL to about 2,500 mL, is from about 0.25 gI to about 40 gI with the volume of the liquid receivable from about 80 mL to about 2,200 mL, is from about 0.3 gI to about 35 gI with the volume of the liquid receivable from about 90 mL to about 2,000 mL, and all values in between.

In some embodiments, the concentrated contrast agent is amidotrizoate, ioxaglic acid, and/or ioxithalamate, and the unit dose of amidotrizoate, ioxaglic acid, and/or ioxithalamate is independently selected from about 5 gI to about 50 gI with the volume of the liquid receivable from about 20 mL to about 300 mL, is from about 10 gI to about 35 gI with the volume of the liquid receivable from about 25 mL to about 250 mL, is from about 11 gI to about 33 gI with the volume of the liquid receivable from about 30 mL to about 200 mL, and all values in between.

In some embodiments, the unit dose and the volume of the liquid receivable for the concentrated contrast agent is selected based on the contrast agent and the procedure to be performed (e.g. X-ray procedures such as computed tomography and/or radiograph, magnetic resonance imaging, and so on).

In an example not part of the invention, the unit dose is a concentrated medicament. In some examples the concentrated medicament is selected from the group consisting of an oral electrolyte, an oral glucose tolerance test (OGTT) composition, nutritional supplements used together with one or more therapeutic or contrast agent, a intestinal lavage solution, a laxatives for colorectal examination or abdominal operation, an agent or composition for improvement of intestinal function or hyperammonemia, and a mucosal protectant or a hemostatic. In some embodiments, the concentrated medicament is free of functional excipients and additives. In some embodiments, the concentrated medicament is in a powder form, in a liquid form, or in a tablet form.

In some examples, the apparatus further includes a barrier member, and the container body and the cap are collectively disposed within an interior of the barrier member. In some embodiments, the container body is configured to be used by a patient to orally consume the concentrated medicament or the concentrated contrast agent through the opening of the container body when the concentrated medicament or the concentrated contrast agent has been diluted with the volume of liquid.

In some embodiments, the container body includes at least a portion that is transparent, and a plurality of indications are disposed on the portion that is transparent. Each indication from the plurality of indications corresponds to a volume of liquid to be received within the interior of the container body, such that a corresponding predetermined dilution of the unit dose of the concentrated medicament and the concentrated contrast agent can be achieved.

In some embodiments, the container body includes at least a portion that is transparent. The apparatus further includes a label disposed on the container body and includes a plurality of indications, each corresponding to a volume of liquid to be received within the interior of the container body such that a corresponding predetermined dilution of the unit dose of the concentrated medicament or the concentrated contrast agent can be achieved. The plurality of indications are disposed on the label adjacent the transparent portion.

In some embodiments, the container body includes at least a portion that is transparent. The apparatus further includes a label disposed on the container body and includes a plurality of indications, each corresponding to a volume of liquid to be received within the interior of the container body and a corresponding concentration level. The plurality of indications are disposed on the label adjacent the transparent portion.

In some embodiments, an apparatus includes a container body defining an opening in fluid communication with an interior of the container body, and a cap coupled to the container body and enclosing the opening. The apparatus also includes a unit dose of a dilutable substance disposed within the interior of the container body. The unit dose of the dilutable substance is configured to be diluted to a select dilution strength with a volume of a liquid receivable through the opening and within the interior of the container body. The apparatus also includes a barrier member defining an interior, where the container body and the cap are collectively disposed within the interior of the barrier member.

The barrier member is configured to shield the unit dose of the dilutable substance from at least one of light or moisture. In some embodiments, the barrier member includes a laminate of polymer and aluminum. In some embodiments, the barrier member is sealed with an ultrasonic seal or a heat seal.

In some embodiments, the apparatus further includes instructions for use disposed within the interior of the barrier member.

The unit dose of a dilutable substance is a unit dose of a contrast agent. In some examples not part of the invention the unit dose of a dilutable substance is a unit dose of a medicament. In some embodiments, the unit dose of a dilutable substance is a unit dose of a contrast agent, and the barrier member is configured to shield the unit dose of the contrast agent from at least one of light or moisture.

In some embodiments, the container body includes at least a portion that is transparent and a plurality of indications are disposed on the portion that is transparent. Each indication from the plurality of indications corresponds to a volume of liquid to be received within the interior of the container body, such that a corresponding predetermined dilution of the unit dose of a dilutable substance can be achieved.

In some embodiments, the container body includes at least a portion that is transparent. The apparatus further includes a label disposed on the container body and includes a plurality of indications, each corresponding to a volume of liquid to be received within the interior of the container body such that a corresponding predetermined dilution of the unit dose of a dilutable substance can be achieved. The plurality of indications are disposed on the label adjacent the transparent portion.

In an example, an apparatus consists essentially of a container body that defines an opening in fluid communication with an interior of the container body. A cap is coupled to the container body and encloses the opening. A unit dose of a dilutable substance is disposed within the interior of the container body. The unit dose of the dilutable substance is configured to be diluted to a select dilution strength with a volume of a liquid that is receivable through the opening and within the interior of the container body. A barrier member defines an interior. The container body and the cap are collectively disposed within the interior of the barrier member. Instructions for use are disposed within the interior of the barrier member. The container body is configured to be used by a patient to orally consume the unit dose of the dilutable substance through the opening of the container body when the unit dose of the dilutable substance has been diluted with the volume of liquid. The barrier member is configured to shield the unit dose of the dilutable substance from at least one of light or moisture.

In an example, an apparatus consists of a container body that defines an opening in fluid communication with an interior of the container body. A cap is coupled to the container body and encloses the opening. A unit dose of a dilutable substance is disposed within the interior of the container body. The unit dose of the dilutable substance is configured to be diluted to a select dilution strength with a volume of a liquid that is receivable through the opening and within the interior of the container body. A barrier member defines an interior. The container body and the cap are collectively disposed within the interior of the barrier member. Instructions for use are disposed within the interior of the barrier member. The container body is configured to be used by a patient to orally consume the unit dose of the dilutable substance through the opening of the container body when the unit dose of the dilutable substance has been diluted with the volume of liquid. The barrier member is configured to shield the unit dose of the dilutable substance from at least one of light or moisture.

In an example, a method includes removing a cap from a container body. The container body has a unit dose of one of a concentrated medicament and a concentrated contrast agent disposed within an interior defined by the container body. The method also includes adding a volume of a liquid to the interior of the container body such that the one of a concentrated medicament and a concentrated contrast agent is diluted to a select dilution strength. The method further includes providing the container body to a patient to orally consume the diluted one of a medicament and a concentrated contrast agent.

In an example the one of a concentrated medicament and a concentrated contrast agent is a contrast agent, and the container body is provided to the patient prior to an imaging procedure to be performed on the patient. In some embodiments, the method further includes, after the adding the volume of liquid, coupling the cap to the container body, and agitating the container body such that the volume of liquid is mixed with the one of a concentrated medicament and a concentrated contrast agent.

In an example the method further includes, after the adding the volume of liquid, coupling the cap to the container body, and agitating the container body such that the volume of liquid is mixed with the one of a concentrated medicament and a concentrated contrast agent. The method also includes removing the cap from the container body prior to providing the container body to the patient.

In an example the method further includes, after the adding the volume of liquid, coupling the cap to the container body and agitating the container body such that the volume of liquid is mixed with the one of a concentrated medicament and a concentrated contrast agent prior to providing the container body to the patient.

In some embodiments, the one of a concentrated medicament and a concentrated contrast agent is a medicament. In some embodiments, the one of a concentrated medicament and a concentrated contrast agent is a contrast agent.

In an example the method further includes, prior to the removing the cap, removing the container body with the cap disposed thereon from an interior of a barrier member. The barrier member is configured to shield the unit dose of the one of a concentrated contrast agent and a concentrated medicament from at least one of light or moisture.

In an example another method includes removing a cap from a container body. The container body has a unit dose of a functional excipient free concentrated contrast agent disposed within an interior defined by the container body. The method also includes adding a volume of a liquid to the interior of the container body such that the unit dose of the functional excipient free concentrated contrast agent is diluted to a select dilution strength. The method further includes providing the container body to a user. In an example, the container body is provided to the patient prior to an imaging procedure to be performed on the patient.

In some embodiments, the method further includes, after adding the volume of liquid, coupling the cap to the container body and agitating the container body, such that the volume of liquid is mixed with the functional excipient free concentrated contrast agent.

In some embodiments, the method further includes, after adding the volume of liquid, coupling the cap to the container body and agitating the container body, such that the volume of liquid is mixed with the functional excipient free concentrated contrast agent. The method also includes removing the cap from the container body prior to providing the container body to the patient.

In some embodiments, the method further includes, after adding the volume of liquid, coupling the cap to the container body and agitating the container body, such that the volume of liquid is mixed with the functional excipient free concentrated contrast agent prior to providing the container body to the patient.

In some embodiments, the method further includes, prior to removing the cap, removing the container body with the cap disposed thereon from an interior of a barrier member. The barrier member is configured to shield the unit dose of the functional excipient free concentrated contrast agent from at least one of light or moisture.

In some embodiments, yet another method includes removing a cap from a container body. The container body has a unit dose of a dilutable powder contrast agent disposed within an interior defined by the container body. The method further includes adding a volume of a liquid to the interior of the container body, such that the unit dose of the powder contrast agent is diluted to a select dilution strength with the volume of liquid receivable through the opening and substantially filling the interior of the container body. The method also includes providing the container body to a patient to orally consume the diluted powder contrast agent. In some embodiments, the container body is provided to the patient prior to an imaging procedure to be performed on the patient.

In some embodiments, the method further includes, after adding the volume of liquid, coupling the cap to the container body and agitating the container body, such that the volume of liquid is mixed with the unit dose of a dilutable powder contrast agent.

In some embodiments, the method further includes, after adding the volume of liquid, coupling the cap to the container body and agitating the container body, such that the volume of liquid is mixed with the unit dose of a dilutable powder contrast agent. The method also includes removing the cap from the container body prior to providing the container body to the patient.

In some embodiments, the method further includes, after adding the volume of liquid, coupling the cap to the container body and agitating the container body, such that the volume of liquid is mixed with the unit dose of a dilutable powder contrast agent prior to providing the container body to the patient.

In some embodiments, the method further includes, prior to removing the cap, removing the container body with the cap disposed thereon from an interior of a barrier member. The barrier member is configured to shield the unit dose of a dilutable powder contrast agent from at least one of light or moisture.

In some embodiments, the unit dose of the dilutable powder contrast agent is a unit dose of dilutable powder iodinated contrast agent. In some embodiments, the iodinated contrast agent is selected from the group consisting of iohexol, iopamidol, ioxilan, iopromide, iotrolan, iodixanol, iopentol, ioversol, iomeprol, iobitridol, amidotrizoate, metrizoate, ioxaglate, ioxaglic acid, and ioxithalamate. In some embodiments, the unit dose of the unit dose of iohexol is from about 0.5 gI to about 25 gI with the volume of the liquid from about 80 mL to about 2,000 mL. In some embodiments, the unit dose of iohexol is from about 0.2 gI to about 75 gI with the volume of the liquid from about 4 mL to about 250 mL. In some embodiments, the unit dose of the dilutable powder contrast agent is a unit dose of dilutable powder iodinated contrast agent that includes an amount of iodine, such that the dilutable powder iodinated contrast agent can be diluted to a dilution strength of less than approximately 4.5 mgI/ml. In some embodiments, the dilutable powder iodinated contrast agent can be diluted to a dilution strength of less than approximately 6 mgI/ml.

As used herein, the term "medicament" refers to a compound or composition which is suitable for oral administration and is used for medical treatment and diagnosis. Examples of the medicament may include, but are not limited to oral electrolytes, oral glucose tolerance test (OGTT) compositions, nutritional supplements used together with one or more therapeutic or contrast agent, intestinal lavage solutions, laxatives for colorectal examination or abdominal operation, agents or compositions for improvement of intestinal function or hyperammonemia, and mucosal protectants or hemostatics. In one embodiment, the medicament does not include an enteral nutrient.

As used herein, a "functional excipient or additive" denotes an excipient and additive that modifies the dissolution properties of the concentrated medicament or concentrated contrast agent, for example, its solubility or dissolution rate. In other words, the dissolution profile of a concentrated medicament or concentrated contrast agent with the functional excipient or additive is moderately or substantially different from the one without the functional excipient or additive. Examples of the function excipients and additives include, but are not limited to, disintegrating agent, dispersants, beta-cyclodextrins and analogs, and etc.

As used herein, the term "non-functional excipient or additive" denotes an excipient or additive that is not a functional excipient or additive, for example, a flouring excipient, a flavoring agent (e.g., a sweetener), a coloring agent, and etc. In other words, the dissolution profile of a concentrated medicament or concentrated contrast agent with the non-functional excipient or additive is the same or substantially the same as the one without the non-functional excipient or additive. A non-functional excipient or additive can have some impact on the release of the medicament or contrast agent due to the initial dissolution, hydration, etc., but would not be considered to be a significant deviation from the one without the non-functional excipient or additive.

As used herein, the term "contrast agent" refers to a substance used to enhance or improve the visibility of internal bodily structures in medical imaging. For example, the contrast agent is often used to enhance the visibility of blood vessels and the gastrointestinal tract. A "radiocontrast agent" refers to a contrast agent used in X-ray imaging techniques, such as computed tomography (CT) and radiography (X-ray imaging). A "magnetic resonance imaging" (MRI) agent refers to a contrast agent used in MRI techniques. A "ultrasonic imaging" agent refers to a contrast agent used in sonographic diagnosis.

As used herein , the term "sterile" when used in connection with a substance (e.g. a unit dose) or an element (e.g. a container body) reflects that the substance and/or element has been treated to be and/or is believed to be substantially free of undesirable microorganisms such as, but not limited to, a virus, a bacteria, and/or the like. As used herein, the term "non-sterile" when used in connection with a substance (e.g. a unit dose) or an element (e.g. a container body) reflects that the substance and/or element has not been treated to be and/or is believed not to be substantially free of undesirable microorganisms such as, but not limited to, a virus, a bacteria, and/or the like.

The term "about" when used in connection with a referenced numeric indication means the referenced numeric indication plus or minus up to 15% of that referenced numeric indication. In one embodiment, term "about" means the referenced numeric indication plus or minus up to 10% of that referenced numeric indication. For example, "about 100" means from 90 to 110.

Unless explicitly noted otherwise, a specification of a medicament or a contrast agent is intended to encompass free base form(s), salt form(s), anhydrous form(s), solvate(s) thereof, and corresponding composition(s) of the specified medicament/contrast agent. For example, the listed concentrated MRI agent gadopentetate encompasses all salt forms (e.g. gadopentetate dimeglumine) as well as the free base form (gadopentetic acid). Similarly, the ionic iodine-based radiocontrast agent ioxaglate encompasses all salt forms (e.g. ioxaglate meglumine) as well as the free base form (ioxaglic acid).

In an example a method of using a container as described herein can include removing a cap from a container body. The container body can have a unit dose of a concentrated medicament or a concentrated contrast agent disposed within an interior defined by the container body. A volume of a liquid can be added to the interior of the container body such that the concentrated medicament or contrast agent is diluted to a select dilution strength. The container body can be provided to a patient to consume orally the diluted medicament or concentrated contrast agent.

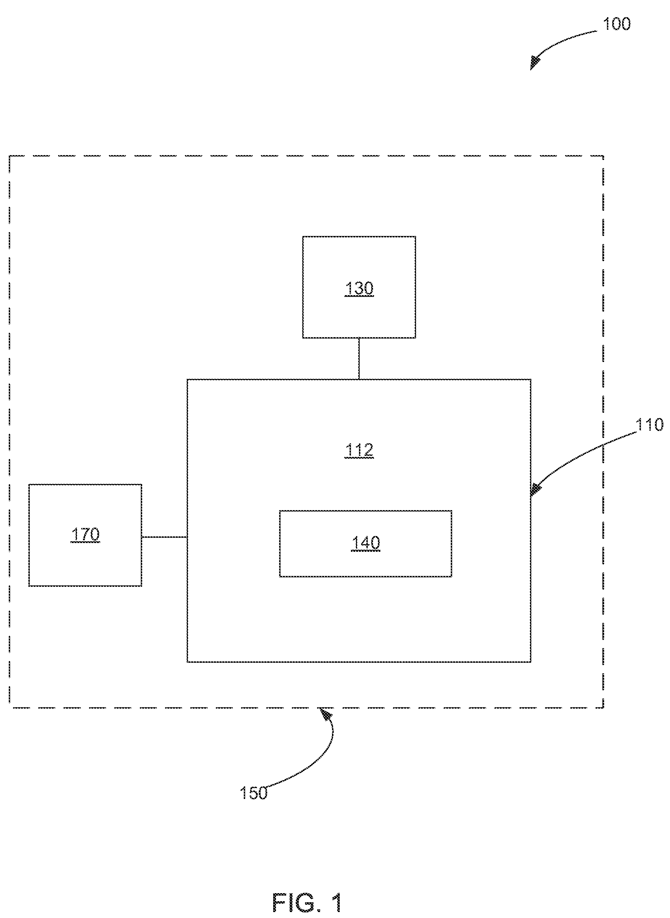

FIG. 1 is a schematic illustration of a container, according to an embodiment. A container 100 can include a container body 110 and a cap 130 that can be removably coupled to the container body 110. The container body 110 defines an interior volume 112 that can contain a concentrated substance 140 and can receive a volume of fluid to dilute the concentrated substance 140, as described in more detail below. The concentrated substance 140 can be, for example, a unit dose of a concentrated medicament or a concentrated oral contrast agent (also referred to as "contrast agent" or "oral contrast media" or "contrast media"). The container 100 can be used to store and transport the concentrated substance 140 to, for example, a healthcare facility. The container 100 can also be used at the healthcare facility to dilute the concentrated substance 140 and to provide the diluted substance to a user (e.g., a patient) for oral consumption.

The container body 110 can be a variety of different shapes, sizes and configurations. For example, the container body 110 can be in the form of a bottle, jar, tub, jug, can, and/or any other type of container. The container body 110 can have a volume of, for example, 355 ml, 500 ml, 1000 ml, 2000 ml, 20 ounces, or any other suitable volume. The container body 110 can, in some embodiments, be formed of plastic material, such as, for example, polyethylene terephthalate (PET), high density polyethylene (HDPE), low density polyethylene (LDPE), acrylic, polypropylene (PP), or any other suitable plastic. In other embodiments, the container body 110 can be formed of glass, aluminum, steel, and/or any other suitable material. The container body 110 can also be formed partially or completely with a transparent material such that the contents (e.g., the concentrated substance 140) of the container body 110 are visible. In some embodiments, the container body 110 can be formed with a material such that the container body 110 is recyclable.

The concentrated substance 140 can be provided in a variety of different forms. For example, the concentrated substance 140 can be a solid (in the form of one or more tablets, or smaller granules, powder, etc., either loose or contained in, for example, capsules or bags), a liquid, and/or any other suitable concentrate form. As described above, the concentrated substance 140 can be a concentrated medicament or a concentrated contrast agent. For example, the concentrated substance 140 can be a radiological contrast agent, such as, an iodinated contrast agent. In some embodiments, the concentrated substance 140 can be, for example, a non-ionic iodinated contrast agent, such as iohexol, iopamidol, ioversol, iopromide, ioxilan, iopentol, iomeprol, iobitridol, iotrolan and/or iodixanol. In some embodiments, the concentrated substance 140 can be, for example, diatrizoate meglumine and/or diatrizoate sodium, metorizoate, ioxaglate, ioxithalamate solution. In some embodiments, the concentrated substance 140 can be, for example, a monoisomer of a medicament or contrast agent, or a mixture of two or more isomers in any ratio.

In some embodiments, the concentrated substance 140 can be provided in a pure or a substantially pure form. For example, the concentrated substance 140 can be free of excipients or additives. For example, the concentrated substance 140 can be a pure or substantially pure powdered (e.g., spray dried, filter dried, milled, sifted, etc.) iohexol with a particle size of, for example, 40-50 microns or less. The dissolvability of the pure or substantially pure powdered iohexol is substantially independent of particle size. Thus, pure or substantially pure powdered iohexol can be provided in the container 100 having any suitable particle size, while still being readily and rapidly dissolvable. In one example, the pure or substantially pure powdered iohexol can be dissolved, for example, when manually agitated for between 10-20 seconds.

In an alternative embodiment, the concentrated substance 140 can be a contrast agent or medicament in the form of granules, i.e. larger particles (for example, up to 200 microns) than a powdered form. Granules may also dissolve relatively quickly, but may be more difficult to process, e.g. to dispense into container body 110 during manufacturing.

The concentrated substance 140 can be, for example, a pre-measured unit dose of a medicament or a contrast agent that can be diluted to a desired concentration strength to be orally consumed by a patient. For example, in some embodiments, the concentrated substance 140 can be approximately 9.7 g of pure or substantially pure iohexol powder that can be diluted with a liquid diluent to a selected dilution strength in the container body 110, and orally consumed from the container body 110 by a patient. In some embodiments, the diluent is selected is any suitable diluent that can provide a stable preparation of the concentrated substance 140 (e.g. an iodinated contrast agent) for the time frame associated with preparing the concentrated substance for consumption via the methods described herein. In some embodiments, the diluent has one or more components that are likely to increase patient compliance. For example, the diluent can include, but is not limited to, one or more of water, a fruit juice, a sports drink, a pediatric/infant formulation, a sweetening agent, a flavoring agent, and/or the like.

In other embodiments, the concentrated substance 140 can be approximately 13g of pure iohexol powder or any other suitable quantity of iohexol. In other embodiments, the concentrated substance 140 can be a formulated concentrate. In some embodiments, a desired dilution strength of iohexol can be, for example, between 4.5 mg/ml (or can alternatively be referred to as 4.5 mgI/mL (grams of Iodine per milliliter)) and 25 mg/ml (or 25 mgI/mL). In some embodiments, a desired dilution strength of iohexol can be, for example, any of 6 mg/ml (or 6 mgI/mL), 9 mg/ml (or 9 mgI/mL), 12 mg/ml (or 12 mgI/mL), 15 mg/ml (or 15 mgI/mL), 18 mg/ml (or 18 mgI/mL), and 21 mg/ml (or 21 mgI/mL). Similarly stated, in some embodiments, the concentrated substance 140 can contain excipients such as dispersants, disintegrants, coatings, enteric, fillers, flavors, glidants, sorbents, preservatives, sweeteners, colors, wetting agents, binders, anti-caking agents, and/or any other suitable substances to enhance dispersal, dilution, stability, taste, processability, absorption, appearance, etc. of the concentrated substance 140.

The concentrated substance 140 can occupy less than the entire interior volume 112 of the container body 110 such that a diluent can be received in the interior volume 112 to dilute the concentrated substance 140 to a desired dilution strength. The diluent can be a liquid such as, for example, water, sugar-water solution, fruit juice, milk, carbonated beverage, and/or any other suitable liquid that can be mixed with the concentrated substance 140 to dilute the concentrated substance 140 to a desired or selected dilution strength. In some embodiments, the diluent can be selected to improve palatability of the concentrated substance 140.

The container body 110 can define an opening (not shown in FIG. 1) in fluid communication (e.g., a fluid, such as a gas or a liquid, can pass between the opening and the interior volume) with the interior volume 112 of the container body 110. The cap 130 can be removably coupled to the container body 110 to close or obstruct the opening. For example, the cap 130 can be threadably coupled to a neck portion (not shown in FIG. 1) of the container body 110. When the cap 130 is removed from the container body 110, a diluent (e.g., a liquid) can be introduced into the interior volume 112 of the container 110 through the opening. The cap 130 can be, for example, a tamper-evident medicament cap, a childproof cap, and/or any other suitable type of cap that can seal the container body 110. In some embodiments, the cap 130 can be resealable. For example, the cap 130 can include one or more liners and/or seals that can form a fluid-tight seal with the container body 110 when the cap 130 is coupled thereto. The cap 130 can be formed with, for example, one or more materials, such as, for example, PP, PET, HDPE, LDPE, aluminum, steel, and/or any other suitable material(s).

The container 100 can optionally include a label 170. The label 170 can be coupled to the container body 110 and provide information, such as, for example, information about the contents of the container 100. The label 170 can be coupled to the container body 110 with, for example, an adhesive. In some embodiments, the label 170 can be coupled to the container body 110 with an adhesive that allows the label 170 to be removable or peelable with limited or no damage to the label 170. For example, it may be desirable to remove the label 170 from the container body 110 and couple the label 170 to another object or item, such as, for example, to a patient record or chart. In other embodiments, the label 170 can be coupled to and/or provided within an exterior packaging, such as a barrier member 150 (described in further detail below), or other exterior packaging.

In some embodiments, such as embodiments in which the concentrated substance 140 is a medicament or contrast agent, the label 170 can contain drug and/or regulatory information. The label 170 can also include use instructions, such as instructions regarding the amount of diluent to add to the container body 110 to obtain a desired dilution strength, instructions regarding the mixing of the concentrated substance 140 and the diluent, and/or instructions related to the consumption of the contents of the container body 110. For example, different dilution strengths may be desired for different types of the concentrated substance 140 and/or for different uses of the concentrated substance 140. For example, a dilution strength for a contrast agent for use in imaging of an upper gastrointestinal (GI) tract may be different than a dilution strength for use of a contrast agent for imaging of a lower portion of the GI tract.

In some embodiments, the label 170 can include one or more markings or indications (not shown in FIG. 1) that can be used by a medical professional or the patient to measure the desired amount of diluent to add to the container body 110 to obtain a desired dilution strength. For example, the label 170 can contain markings located adjacent to a transparent portion of the container body 110. The markings can include, for example, volumetric measurement graduations such as graduations in milliliters (ml), or ounces, and/or the markings can include concentration measurement graduations, such as graduations in milligrams per milliliter (mg/ml) (e.g., milligrams of concentrated substance and/or active ingredient per milliliter), etc. In some embodiments, the markings can include a combination of different types of markings or indications. For example, the label can include markings associated with fill volume, for example, in milliliters, and parallel markings associated with concentration strengths, for example, in milligrams per milliliter. In some embodiments, the markings can be indicators that identify one or more fill levels that correspond to one or more specified dilution strengths rather than actual measurement graduations (e.g., ml). The use of the marking or indications can permit the dilution of the same quantity of the concentrated substance 140 to different strengths/concetrations/volumes. Similarly stated, the same quantity of the concentrated substance 140 (which can be characterized as a unit dose, in some embodiments) can be administered across different recipients regardless of the dosing protocol, and regardless of the volume of diluent employed.

In some embodiments, instructions can be provided that instruct the user to add diluent to a particular indicator that corresponds to a particular dilution strength (e.g., mg/ml). In other embodiments, the label 170 can include directions instructing a user to add a volume of diluent to the container body 110 to obtain a specified dilution strength and/or amount of diluted concentrated substance 140. For example, the label 170 can include directions to instruct a user to add a pre-measured volume of diluent to the container body 110 based on the amount of concentrated substance 140 disposed within the container body 110 such that a desired volume and concentration strength of the diluted concentrated substance 140 can be obtained for consumption by a user. In an alternative embodiment, the container body 110, rather than the label 170, can include markings or indications that can be used to prepare the concentrated substance 140 for consumption by a user. For example, the container body 110 can have markings imprinted, formed, molded, engraved, etc., into the material of the container body 110, or the markings can be engraved or printed on the container body 110. In some embodiments, the label 170 can include a portion that can be used by a medical professional or a patient to add a notation(s), such as, for example, the patient name, type of drug, date of preparation, dosage administered, etc. In some embodiments, both the container body 110 and the label 170 can include markings.

In some embodiments, the container body 110 can be stored and/or transported within an optional barrier member 150. The barrier member 150 can be a variety of different shapes, sizes and/or configurations. For example, the barrier member 150 can be a pouch or bag, a box, or other suitable packaging form. The barrier member 150 can be formed with, for example, a material or materials that can reduce or eliminate exposure of the concentrated substance 140 to moisture and/or light. For example, the barrier member 150 can be, formed with, for example, a polymer-aluminum laminate. The barrier member 150 can be, for example, hermetically sealed via ultrasonic welding, heat sealing, and/or any other suitable sealing mechanism. Such a barrier member 150 can be desirable, for example, for storage and/or transport of certain concentrated medicaments and/or contrast agents, which are sensitive to light and/or moisture. For example, substances such as a pure or substantially pure iohexol can be sensitive to moisture and/or light. For example, exposure to moisture over a time period can result in clumping or agglomeration of the particles of iohexol, which can affect the effectiveness and/or usability of the iohexol. In some cases, agglomeration can occur, for example, in several days to several weeks. Thus, if the container body 110 is formed with a material that is not sufficiently impermeable to light and/or moisture (e.g., certain PET materials) and contains a concentrated substance such as a hygroscopic monoisomer of iohexol, it may be desirable to place the container 100 within such a barrier member 150.

In other embodiments, the container body 110 can be formed with a material that is sufficiently impermeable to moisture and/or light such that the concentrated substance 140 is sufficiently protected by the container alone. In such an embodiment, the container 100 can be stored and transported without a separate barrier member 150. For example, in some embodiments, the container body 110 can be formed with an opaque material and/or a material with low permeability, such that the container body 110 can prevent light and/or moisture from passing through the container body 110. In some embodiments, in addition to the container body 110 and/or a barrier member 150, or alternatively, the label 170 can provide protection to the concentrated substance 140 within the container body 110. For example, the label 170 can be opaque to prevent light from passing through the container body 110 and/or the label 170 can include a low-permeability component, such as a foil backing. The label 170 can be bonded to the container 110 such that exposure of the concentrated substance 140 to light and/or moisture can be reduced or eliminated. For example, the label 170 can be sized such that, when applied to the container body 110, the label 170 covers substantially all or a portion of the surface of the container body 110.

As described above, the container 100 can be used to store and transport the concentrated substance 140 (e.g., a concentrated medicament or contrast agent) and can also be used during the dilution, mixing and consumption of the concentrated substance 140. Thus, the use of other containers and/or measuring devices can be reduced or eliminated. For example, the container 100 can be delivered to a medical facility and a medical professional can remove the cap 130 from the container body 110 and add a desired amount of diluent to the container body 110 and then replace the cap on the container body 110 to reseal the container body 110. The user can obtain the desired dilution strength by adding the diluent to an appropriate marking on the label 170 or container body 110, as described above. The medical professional can then agitate (e.g., either manually or mechanically) the container 100 such that the diluent is mixed with the concentrated substance 140 and dilutes and/or dissolves the concentrated substance 140. For example, as described above, when the concentrated substance 140 is a pure or substantially pure powder iohexol, the concentrated substance 140 can be readily and rapidly dissolved through manual agitation by a user. The medical professional can then provide the container 100 to a patient (with or without the cap 130 coupled thereto) so that the patient can orally consume the diluted substance using the container body 110. For example, in some embodiments, the concentrated substance 140 can be a concentrated contrast agent, and the diluted contrast agent can be provided to a patient for oral consumption prior to an imaging procedure.

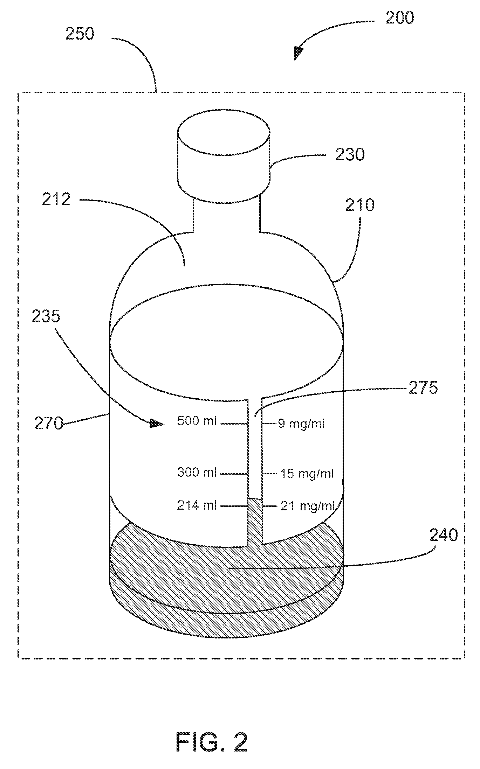

FIGS. 2-4 illustrate a container, according to another embodiment. A container 200 includes a container body 210 and a cap 230 that can be removably coupled to the container body 210. The container 200 can be functionally and/or structurally the same as or similar to the container 100 described above with reference to FIG. 1. The container body 210 defines an interior volume 212 and an opening 220 in fluid communication with the interior volume 212. A concentrated substance 240 is disposed within the interior volume 212 of the container body 210. The cap 230 can include a seal and/or liner to hermetically seal the interior volume 212 and include a threaded portion (not shown) that can be threadably coupled to a threaded portion 236 of the container body 210. The container 200 can also include a label 270 coupled to the container body 210.

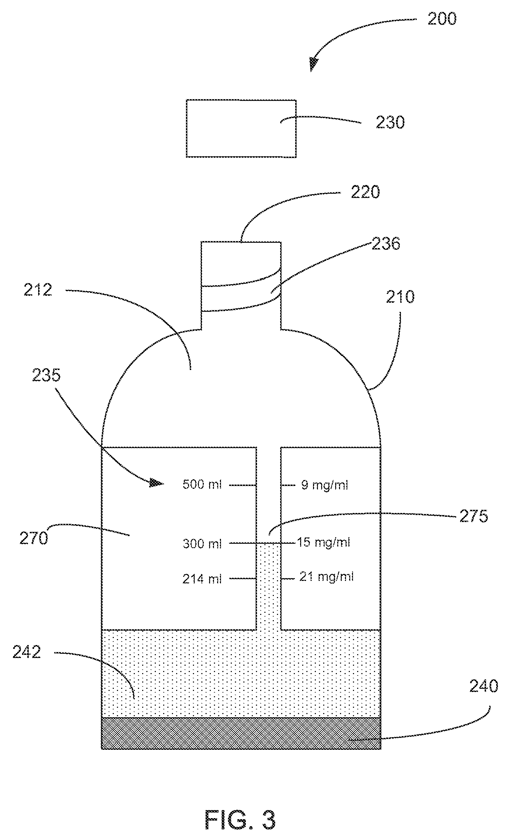

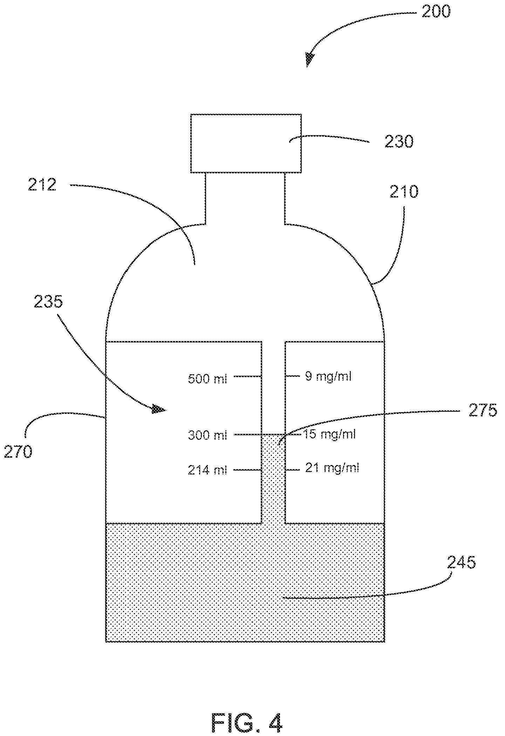

FIG. 2 depicts the container 200 in a first configuration, during storage and/or transport, in which the cap 230 is coupled to the container body 212 and the container 200 is disposed within a barrier member 250. FIG. 3 depicts the container 200 in a second configuration, in which the cap 230 is removed from the container body 210 and a diluent 242 (e.g., water or flavored liquid) has been added to the container body 210, and FIG. 4 depicts the container 200 in a third configuration, in which the concentrated substance 240 has been diluted or dissolved to form a diluted substance 245.

In this embodiment, the concentrated substance 240 is in the form of a concentrated powder. As shown in FIG. 2 and FIG. 3 the quantity of concentrated substance 240 is such that the interior volume 212 includes sufficient head space to allow for the addition of a volume of diluent 242 (e.g., water) to dilute or dissolve the concentrated substance 240 to a select dilution strength, as described in more detail below. The concentrated substance 240 can be, for example, a unit dose of a concentrated medicament or a concentrated contrast agent as described above.

The barrier member 250 can be, for example, in the form of an aluminum-polymer laminar bag in which the container 200 can be disposed during storage and transport as described above with reference to FIG. 1. The barrier member 250 can sealably enclose the container 200 to shield and/or protect the container 200 and its contents (e.g., concentrated substance 240) from moisture and/or light. For example, as shown in FIGS. 2-4, the container body 210 can be formed with a transparent material and the barrier member 250 can prevent the concentrated substance 240 from being exposed to light.

The label 270 can be sized such that a window 275 is defined through which a user (e.g., a medical professional or patient) can view a fill level of the interior volume 212 as the diluent 242 is added to the container body 210. The label 270 can include one or more measurement markings or indications 235 that can be used to measure an amount of diluent to add to the container body 210 to obtain a desired dilution strength and/or volume of a diluted substance 245. The markings 235 can include, for example, volumetric graduations such as graduations in milliliters, ounces, etc., and concentration levels corresponding to the volumetric graduations as shown in FIGS. 2-4, and/or other indicators. The type and quantity of markings 235 shown in FIGS. 2-4 are just an example of the type and quantity of markings that can be used and are not to scale. It should be understood that other types and quantities of markings can alternatively be used. For example, the type and quantity of markings can depend on the particular concentrated substance 240.

In use, a user (e.g., healthcare professional or patient) can open the barrier member 250 (e.g., cut or tear the bag or pouch) and remove the container 200 from the barrier member 250. The cap 230 can then be removed from the container body 210, exposing the opening 220. The user can then add a volume of diluent 242 through the opening 220 and fill the container body 210 to a desired marking 235 as shown in FIG. 3. In this example, the volume of liquid has been added to the marking 300 ml, which in this example corresponds to a concentration of 15 mg/ml. In other embodiments, the user can add a different volume of diluent to the container body 210 to obtain a different concentration strength. The user can then recouple the cap 230 to the container body 210 and agitate the container 200 to mix the diluent 242 and the concentrated substance 240. After the concentrated substance 240 has been diluted it will be the diluted substance 245, as shown in FIG. 4. The container 200 can then be provided to a patient, either with or without the cap 230 coupled to the container body 212, so that the patient can consume the diluted substance 245 orally. For example, in some embodiments, the concentrated substance is a unit dose of a concentrated contrast agent (e.g., iohexol), and the diluted contrast agent can be provided to a patient to consume orally prior to an imaging procedure.

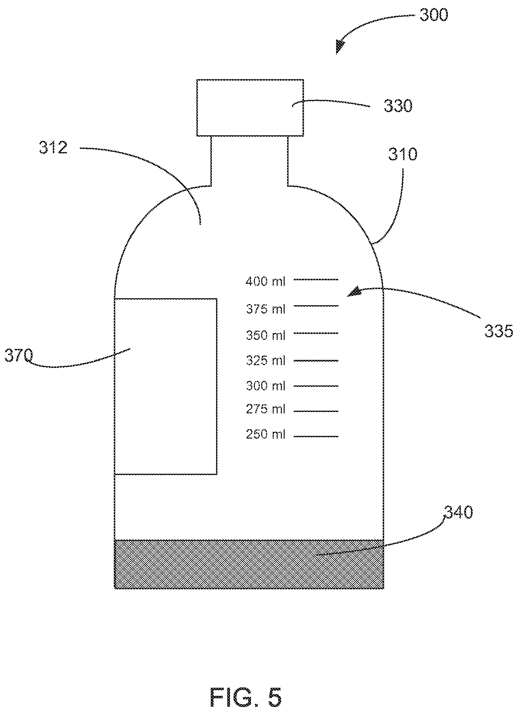

FIG. 5 illustrates a container, according to another embodiment. A container 300 includes a container body 310 and a cap 330 that can be removably coupled to the container body 310 in the same or similar manner as described above for previous embodiments. The container 300 can be functionally and/or structurally the same as, or similar to, the containers 100 and 200 described above. The container body 310 defines an interior volume 312 that can contain a concentrated substance 340 therein and an opening (not shown in FIG. 5) in fluid communication with the interior volume 310. In this embodiment, the concentrated substance 340 is in the form of a concentrated liquid that can be mixed and diluted with the addition of a diluent. The concentrated substance 340 can be for example, a concentrated medicament or a concentrated oral contrast agent. The container 300 also includes a label 370 that can include information about the concentrated substance 340 and directions for its use, as described previously. Although not shown, the container 300 can also be disposed within a barrier member during transport of the container 300 to protect and/or shield the container 300 and its contents from moisture and/or light, as described above for previous embodiments.

In this embodiment, the container body 310 can be formed with a transparent material as with the previous embodiment, and can include multiple markings or indications 335 that can be used to measure the desired amount of diluent to add to the container body 310. For example, the markings 335 can be molded into the material in which the container body 310 is formed, or can be printed on the container body 310, or coupled to the container body 310 by other methods. The container 300 can be used in the same manner as described above for previous embodiments, to store and transport a concentrated substance (e.g.. a concentrated medicament or contrast agent), dilute the concentrated substance to a desired dilution strength, and to be provided to a patient to consume the diluted substance orally.

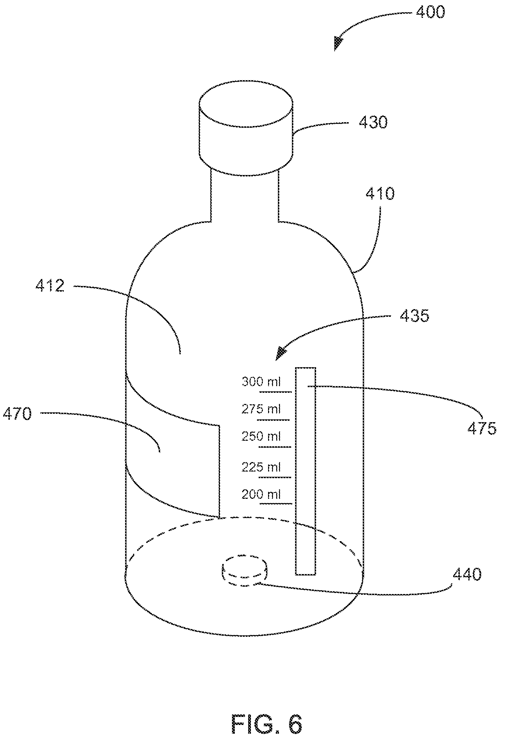

FIG. 6 illustrates a container, according to another embodiment. A container 400 includes a container body 410 and a cap 430 that can be removably coupled to the container body 410 in the same or similar manner as described above for previous embodiments. The container 400 can be functionally and/or structurally the same as, or similar to, the containers 100, 200 and 300 described above. The container body 410 defines an interior volume 412 that can contain a concentrated substance 440 therein and an opening (not shown in FIG. 6) in fluid communication with the interior volume 412. In this embodiment, the concentrated substance 440 is in the form of a tablet that can be dispersed and/or dissolved by the addition of a diluent. The container 400 also includes a label 470 that can include information about the concentrated substance 440 and directions for its use, as described previously.

In this embodiment, the container body 410 is formed with a material that can protect or shield the concentrated substance from moisture and/or light without the use of a separate barrier member (e.g., 150, 250 described above). For example, the container body 410 can be formed with an opaque material having low permeability, for example, a high density polyethylene (HDPE), or a low density polyethylene (LDPE) material. The container body 410 includes a window 475 to allow a user (e.g., healthcare professional) to view at least a portion of the interior volume 412 of the container body 410. For example, the window 475 can be a transparent portion of the container body 410. In alternative embodiments, the container body may be formed with a low permeability material that provides sufficient visibility through the material of the container body to view the contents such that the container body does not need a window. The container body 410 also includes one or more markings or indications 435 that can be used to measure an amount of diluent to add to the container body 410 as described above. Thus, a user can add a volume of diluent to the container body 410 and view the level of the diluent being added through the window 475 and use the markings 435 to measure the desired amount of diluent. The container 400 can be used in the same manner as described above for previous embodiments, to store and transport a concentrated substance (e.g., a concentrated medicament or contrast agent), dilute the concentrated substance to a desired dilution strength, and to be provided to a patient to orally consume the diluted substance.

In an alternative example the concentrated substance can be contained within the cap of the container, rather than in the interior volume defined by container body. For example, the cap can define a compartment or interior region that can contain the concentrated substance. For example, in such an embodiment, the cap can include a seal that can be punctured or removed by the user to allow the concentrated substance to be expelled out of the interior region of the cap and into the interior volume of the container body. The container can then be used in the same manner as described above for other embodiments to add a volume of diluent to dissolve or dilute the concentrated substance within the container body and such that a patient can orally consume the diluted substance using the container body.

In an alternative example the concentrated substance can be contained in an inner container, such as a packet, bag, sachet, etc. that is permeable to the diluents and to the diluted substance. In such an embodiment, when the diluent is added to the container, it permeates the inner container, diluting the concentrated substance. The diluted substance can be released from the inner container.

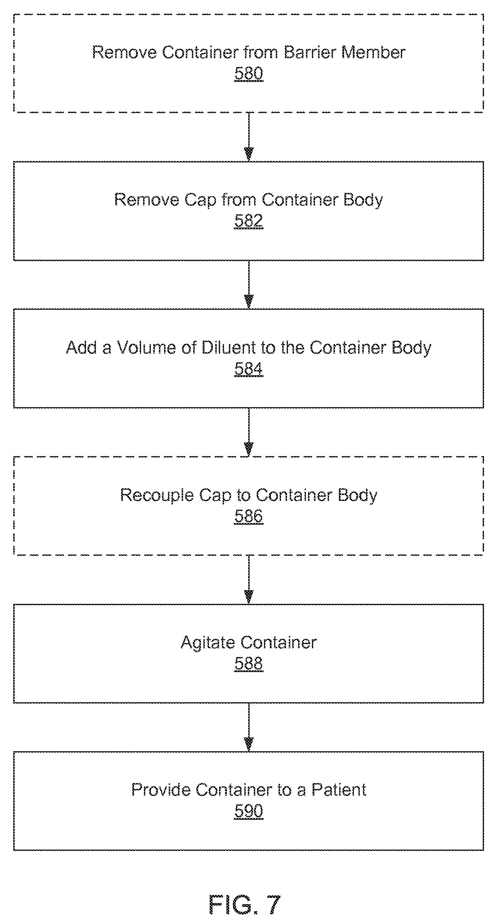

FIG. 7 is a flowchart illustrating a method of using a container including a concentrated substance, such as, for example, the containers (e.g., 100, 200, 300, 400) described herein. Optionally at 580, a user can remove a container (e.g., 100, 200, 300, 400) from a barrier member (e.g., 150, 250). For example, the container can be disposed within a barrier member as described herein that can protect or shield the concentrated substance from being exposed to light and/or moisture. In an embodiment in which the barrier member is in the form of a bag or pouch, the user can, for example, cut or tear the bag or pouch and remove the container. The container can include a container body (e.g., 110, 210, 310, 410) and a cap (e.g., 130, 230, 330, 430) coupled thereto enclosing an interior of the container body as described herein. The container body can have a unit dose of a concentrated substance, such as, for example, a concentrated medicament or a concentrated contrast agent, disposed within its interior.

The user can remove the cap from the container body at 582, exposing an opening defined by the container body. At 584, the user can add a volume of a diluent to the container body via the opening. For example, the user can add a diluent, such as water, to the container to dilute the concentrated substance to a select dilution strength. For example, as described herein, instructions on how to dilute the concentrated substance can be provided with the container. In some embodiments, the instructions can be provided on a label coupled to the container body. In some embodiments, the container body can include one or more markings or indications to use to measure the volume of diluent to be added. The markings can be provided, for example, on the label or on the container body.

After adding the volume of diluent (e.g., water) to the container body, at 586, the user can optionally replace the cap, thereby sealing the container body. At 588, the user can agitate the container, for example, by manually shaking or agitating the container. Agitating the contents can aid in the dispersal and/or dissolution of the concentrated substance into the diluent to provide a homogenous diluted substance. At 590, the container can be provided to a patient so that the diluted substance can be orally consumed by the patient. The container can be provided to the patient with or without the cap couple to the container body. For example, in some embodiments, the concentrated substance can be a concentrated contrast agent, and the diluted contrast agent can be provided to a patient prior to an imaging procedure.

In some examples, one or more substance(s) medicament(s), agent(s), and/or dose(s) as described in conjunction with the apparatuses and methods disclosed herein comprises any suitable formulation(s), such as of the types described herein.

### ORAL REHYDRATION SALTS

In some examples, the apparatuses and methods described herein are usable with an oral rehydration salt to counter dehydration. Similarly stated, in some embodiments, an apparatus as described herein contains any suitable oral rehydration salt and has one or more indications pertinent for reconstituting and/or administering the oral rehydration salt contained within.

Oral rehydration salts (ORS) are orally administered, typically in the form of a reconstituted solution, to counteract dehydration. A single serving (e.g. as might be commercially available in a sachet) is added to water or lukewarm water of a desirable volume (e.g. approximately 100 mL) and is stirringly dissolved at or near the time of use. The oral rehydration solution thereby formed can be administered orally. Aspects of this disclosure permit a unit dose of the ORS to be disposed in an apparatus as described herein, and an oral electrolyte solution can be prepared when needed in accordance with the methods described herein. In this manner, local reconstitution of the ORS lowers transportation costs by reducing the weight to be transported, with the only requirement during reconstitution being safe drinking water.

In some examples the ORS includes at least glucose and sodium. In some embodiments, the ORS includes at least glucose (e.g. in an anhydrous form), sodium (e.g. in the form of sodium chloride), potassium (e.g. in the form of potassium chloride), and dihydrate. In some embodiments, the ORS conforms to regulatory and/or recommended standards, including, but not limited to, those established by the World Health Organization (WHO), the Food and Drug Administration (FDA), and/or the like.

In some examples the oral rehydration solution includes components similar to those described herein in a total concentration of about 200 mmol/L, of about 210 mmol/L, of about 220 mmol/L, of about 230 mmol/L, of about 240 mmol/L, of about 250 mmol/L, of about 260 mmol/L, of about 270 mmol/L, of about 280 mmol/L, of about 290 mmol/L, of about 300 mmol/L, of about 310 mmol/L, and all values in between. In some embodiments, the oral rehydration solution includes glucose in a concentration of about 1 g/L, of about 2 g/L, of about 5 g/L, of about 10 g/L, of about 15 g/L, of about 16 g/L, of about 17 g/L, of about 18 g/L, of about 19 g/L, of about 20 g/L, and all values in between. In some embodiments, the oral rehydration solution includes sodium, such as in the form of sodium chloride (NaCl), in a concentration of about 3.5 g/L of NaCl, of about 4 g/L, of about 4.5 g/L, of about 5 g/L, of about 5.26 g/L, and all values in between. In some examples, the quantity of glucose is the same or higher than the quantity of sodium in the oral rehydration solution. In some embodiments, the quantity of glucose is the same or higher than the quantity of sodium, and the concentration of glucose does not exceed 20 g/L in the oral rehydration solution. In some embodiments, an apparatus containing an ORS as described herein has one or more indications formed thereon selected from about 50 mL, about 100 mL, about 150 mL, about 200 mL, and all values in between. In some embodiments, an apparatus containing an ORS as described herein has a single indication formed thereon of about 100 mL. In some embodiments, the ORS can include one or more additives such as but are not limited to, sweeteners (e.g. sucralose) and flavoring agents.

In some examples the reconstituted ORS (or the oral rehydration solution) has glucose in an amount of about 13.5 g/L. In some embodiments, the oral rehydration solution has dihydrate in an amount of about 2.9 g/L. In some embodiments, the oral rehydration solution has sodium chloride in an amount of about 2.6 g/L. In some embodiments, the oral rehydration solution has potassium chloride in an amount of about 1.5 g/L.

Exemplary ORS that can be employed can include commercially available ORS. In some examples, one or more additives can be added to the ORS prior to, during, or after reconstitution of the ORS.

As a non-limiting example, Table 1 lists Oral Rehydration Salt compositions A and B that are within the scope of being used with aspects of the apparatuses and methods described herein. Table 1 also lists various additives that can be added to each ORS, and possible/recommended amounts associated therewith. In some examples, such compositions can be reconstituted with approximately 100 mL of water using the apparatuses and methods described herein.

**Table 1**

| | Composition A | Composition B |
|---|---|---|
| **Packet Components** | Per packet (4.0 g) | Per packet (4.0 g) |
| sodium chloride | 58 mg | 175 mg |
| potassium chloride | 149 mg | 149 mg |
| anhydrous sodium dihydrogenphosphate | 60 mg | 120 mg |
| sodium citrate hydrate | 196 mg | 196 mg |
| magnesium carbonate | 14mg | 14 mg |

| **Additives** | | |
|---|---|---|
| sucrose | proper quantity | proper quantity |
| glucose | | 160 mg |
| citric acid hydrate | 100 mg | 100 mg |
| flavor | trace amount | trace amount |
| Ethylvanillin | trace amount | - |
| vanillin | trace amount | - |
| propylene glycol | trace amount | - |

### NUTRITIONAL SUPPLEMENTS

In some examples, the apparatuses and methods described herein are usable with a nutritional supplement. Similarly stated, in some embodiments, an apparatus as described herein contains any suitable nutritional supplement and has one or more indications pertinent for reconstituting and/or administering the nutritional supplement contained within.

In some examples the nutritional supplement includes glucose. In some embodiments, an apparatus as described herein contains a nutritional supplement having about 50 grams of glucose, about 60 grams, about 70 grams, about 80 grams, about 90 grams, about 100 grams of glucose, and all values in between. In some embodiments, the apparatus containing the nutritional supplement has one or more indications formed thereon selected from about 50 mL, about 100 mL, about 150 mL, about 200 mL, about 250 mL, about 300 mL, and all values in between. In some embodiments, the nutritional supplement contains one or more additives such as preserving agents, sweetening agents, flavoring agents, and/or the like.

As a non-limiting example, an apparatus as described herein can contain a required, recommend, and/or any other amount of glucose (e.g. about 75 g), and can also optionally contain preserving agents (e.g. citric acid) and/or flavoring agents. Indications on the apparatus can be set to any suitable level depending on the desired final concentration of the nutritional supplement (e.g. indications of 75 mL, 150 mL and/or 225 mL for glucose). At the time of use, for oral nutritional supplement, the appropriate water or carbonated water is added to the bottle for dissolution/reconstitution of the nutritional supplement, which can be subsequently orally ingested.

### ORAL GLUCOSE TOLERANCE TEST (OGTT)

In some examples, the apparatuses and methods described herein are usable with an OGTT formulation. Similarly stated, in some embodiments, an apparatus as described herein contains any suitable OGTT formulation and has one or more indications pertinent for reconstituting and/or administering the OGTT formulation contained within.

OGTT formulations are administered for the purpose of determining how quickly orally administered glucose is cleared from the bloodstream of the patient under study. Typically, adults are administered 75 grams of glucose per WHO guidelines irrespective of body weight, and children are administered 1.75 grams per kilogram of body weight. In another variant of the test, administered during pregnancy, administration of 50 grams of glucose over an hour is employed to screen for gestational diabetes.

In some examples the OGTT formulation includes glucose. In some embodiments, the OGTT formulation includes partial hydrolysate of starch. In some embodiments, an apparatus as described herein contains a nutritional supplement having about 40 grams of glucose, about 50 grams, about 60 grams, about 70 grams, about 80 grams, about 90 grams, about 100 grams of glucose, and all values in between. In some embodiments, the apparatus containing the nutritional supplement has one or more indications formed thereon selected from about 50 mL, about 100 mL, about 150 mL, about 200 mL, about 250 mL, about 300 mL, and all values in between. In some embodiments, the nutritional supplement contains one or more additives such as preserving agents, sweetening agents, flavoring agents, and/or the like.

In some examples an apparatus as disclosed herein can contain approximately 75 grams of glucose, and have indications thereon at least for 150 mL and 225 mL. Accordingly, for OGTT in adults ("75 g-OGTT"), a glucose solution of 75 g/225 mL can be prepared with water or carbonated water as described herein. For OGTT during pregnancies as described earlier ("50 g-OGTT"), a glucose solution of 75 g/225 mL can be prepared as described herein, followed by either a) removal of the prepared solution to another container, such that the apparatus contains 150 mL of the glucose solution, and thereby contains 50 g/150 mL, or b) of the apparatus contains an indication of 75 mL, transfer enough glucose solution to another container where 75 mL remains in the apparatus, thereby obtaining 50 g/ 150 mL of the glucose solution in the other container. In this manner, two kinds of OGTT (50 g-OGTT and 75 g-OGTT) can be performed by one product. It is understood that depending on the amount of glucose in the apparatus and the indications on the apparatus, other values of the glucose amounts and/or concentrations are within the scope of this disclosure. It is further understood that substances other than glucose can be employed. In some embodiments, one or more additives such as a corrigent (e.g. citric acid hydrate) and/or flavoring agents can also be part of the OGTT composition.

For example, partial hydrolysate of starch can be employed, as is found in commercially availably OGTT products Compositions C and D.

**Table 2**

| | Composition C | Composition D |
|---|---|---|
| Component(s) | per bottle (150 mL) | per bottle (225 mL) |
| Partial hydrolyzate of starch | 66.7g (50.0 g) | 100.0g (75.0 g) |

| Additive(s) | | |
|---|---|---|
| citric acid hydrate (corrigent) | 0.3 g | 0.45 g |
| flavor | trace amount | trace amount |
| citric acid hydrate | proper quantity | proper quantity |

### INTESTINAL LAVAGE SOLUTION

In some examples, the apparatuses and methods described herein are usable with an intestinal lavage formulation. Similarly stated, in some embodiments, an apparatus as described herein contains any suitable intestinal lavage formulation and has one or more indications pertinent for reconstituting and/or administering the intestinal lavage formulation contained within.

Intestinal lavage can refer to one or more procedures whereby feces, bacteria, mucus, pus, fermentation and putrefaction products, and intestinal excreta are removed from the intestine using an intestinal lavage solution. While some procedures involve introduction of the intestinal lavage solution anally, other procedures employ oral formulations. The orally administered liquid formulation is typically reconstituted from a powder-like substance. Accordingly, aspects of the apparatuses and methods described herein can be employed for preparing intestinal lavage solutions from powder forms of an intestinal lavage formulation. In some embodiments, the intestinal lavage formulation includes one or more electrolytes such as, but not limited to, sodium chloride, potassium chloride, sodium bicarbonate, sulphuric acid, and/or the like. In some embodiments, the intestinal lavage formulation includes one or more additives such as, but not limited, to, laxative agents (e.g. a macrogol such as polyethylene glycol 4000), sweetening agents (e.g. saccharin-based sweetener such as saccharin sodium hydrate), and flavoring agents. In some embodiments, the volume of the apparatus used to prepare an intestinal lavage solution as described herein is about 500 mL, is about 1 L, is about 1.5 L, is about 2 L, is about 2.5 L, is about 3 L, and all values in between.

As a non-limiting example, Table 3 lists intestinal lavage solution compositions that are within the scope of being used with aspects of the apparatuses and methods described herein. Such compositions can be reconstituted with approximately 2 L of water using the apparatuses and methods described herein.

**Table 3**

| Ingredient | Quantity (g)/sachet (137.155 g) |
|---|---|
| NaCl | 2.93 |
| KCl | 1.485 |
| NaHCO₃ | 3.37 |
| H₂SO₄ | 11.37 |

| Additive | |
|---|---|
| PEG 4000 | |
| Saccharin Sodium Hydrate | Unspecified |
| Flavor | |

### LAXATIVES FORMULATIONS

In some examples, the apparatuses and methods described herein are usable with a laxative formulation, such as for colorectal examination and/or abdominal operation. Similarly stated, in some embodiments, an apparatus as described herein contains any suitable laxative formulation and has one or more indications pertinent for reconstituting and/or administering the laxative formulation contained within.

In some examples, the laxative formulation includes magnesium citrate as a laxative drug. In some embodiments, the laxative formulation includes a laxative drug in an amount of about 27 grams, of about 30 grams, of about 34 grams, of about 40 grams, of about 50 grams, of about 60 grams, of about 65 grams, of about 68 grams, and all values in between. In some embodiments, an apparatus containing the laxative formulation has indications formed thereon of about 180 mL, of about 200 mL, of about 300 mL, of about 400 mL, of about 500 mL, of about 700 mL, of about 900 mL, of about 1100 mL, of about 1300 mL, of about 1500 mL, of about 1600 mL, of about 1700 mL, of about 1800 mL, and all values in between. In some embodiments, the laxative formulation contains one or more excipients.

In some examples the apparatus containing the laxative formulation has indications of at least 180 mL and 1,800 mL. In some embodiments, before use for laxative purposes, water is added to the apparatus containing the laxative formulation and a laxative solution of 180 mL or of 1,800 mL is prepared. In some embodiments, an 1,800 mL laxalative solution containing 68 grams of magnesium citrate can be prepared using the apparatuses and methods described herein.

Improvement of Intestinal Function or Treatment of Hyperammonemia

In some examples the apparatuses and methods described herein are usable with formulations for improving intestinal function and/or treating hyperammonemia. Similarly stated, in some embodiments, an apparatus as described herein contains any suitable formulation for improving intestinal function and/or treating hyperammonemia and has one or more indications pertinent for reconstituting and/or administering the formulation for improving intestinal function and/or treating hyperammonemia contained within.

In some examples the formulation is useful for treating neuropsychiatric disorder, finger shivering, abnormal electricencepharogram accompanied by hyperammonemia, postoperative acceleration of exhaust gas or evacuation in obstetrics and gynecology, and improvement of constipation in pediatrics.

In some examples the formulation includes one or more of drugs selected from lactulose, lactitol, and/or the like. In some embodiments, the formulation includes a drug in an amount of about 18 grams, of about 20 grams, of about 24 grams, of about 28 grams, of about 30 grams, of about 32 grams, of about 36 grams, of about 40 grams, and all values in between. In some embodiments, an apparatus containing the laxative formulation can have one or more indications formed thereon selected from about 50 mL, of about 100 mL, of about 150 mL, of about 200 mL, of about 250 mL, of about 300 mL, of about 350 mL, of about 400 mL, and all values in between. In some embodiments, an apparatus containing the laxative formulation has indications formed thereon of about 100 mL, of about 200 mL, and of about 300 mL. In some embodiments, the formulation for improving intestinal function and/or treating hyperammonemia contains one or more excipients.

As a non-limiting example, Table 4 lists laxative compositions (E and F) that are within the scope of being used with aspects of the apparatuses and methods described herein. Such compositions can be reconstituted with approximately 300 L of water using the apparatuses and methods described herein. For example, an apparatus as described herein can contain 39 grams of crystallized lactulose, which can be dissolved in 300 mL of water, and can be consumed by a patient in three steps of 100 mL over the course of a day.

**Table 4**

| Product | Composition | Daily dose (g) |
|---|---|---|
| Composition E | Lactitol hydrate of 6 g is included in a sachet (6 g) | 18 -36 |
| Composition F | Crystallized lactulose of 1,000 mg is included per API of 1g | 19.5 - 39.0 |

### MUCOSAL PROTECTANTS AND/OR HEMOSTASIS

In some examples the apparatuses and methods described herein are usable with mucosal protectant and/or hemostasis formulations. Similarly stated, in some embodiments, an apparatus as described herein contains any suitable mucosal protectant and/or hemostasis formulation and has one or more indications pertinent for reconstituting and/or administering the mucosal protectant and/or hemostasis formulation contained within.

In some examples the mucosal protectant and/or hemostasis formulation includes sodium alginate. In some embodiments, the mucosal protectant and/or hemostasis formulation includes a drug in an amount of about 0.1 gram, of about 0.5 gram, of about 1 gram, of about 1.5 grams, of about 2 grams, of about 2.5 grams, of about 3 grams, of about 3.5 grams, and all values in between. In some embodiments, an apparatus containing the mucosal protectant and/or hemostasis formulation can have one or more indications formed thereon selected from about 10 mL, of about 15 mL, of about 20 mL, of about 30 mL, of about 40 mL, of about 50 mL, of about 60 mL, of about 70 mL, and all values in between. In some embodiments, an apparatus containing the mucosal protectant and/or hemostasis formulation has indications formed thereon of about 20 mL, of about 30 mL, and of about 60 mL. In some embodiments, the mucosal protectant and/or hemostasis formulation contains one or more excipients selected from copper chlorophyll sodium, sodium dehydroacetate, paraben, saccharin sodium (sweetening agent), ethanol and flavoring agents.

In some examples, the mucosal protectant and/or hemostasis formulation present in an apparatus as described herein includes 3 grams of sodium alginate. The apparatus can have indications of about 20 mL, 30 mL and 60 mL. During using, 60 mL of water is added to the apparatus and the sodium alginate is dissolved. The resulting volume can be divided and ingested. In some embodiments 3-4 equal sized portions of the resulting volume are made for purposes of mucosal protection and/or treating hemostasis. In some embodiments, from the same apparatus a single portion of 10 - 30 mL can be isolated for purposes of treating bleeding caused by gastric biopsy, which requires a sodium alginate dose of about 0.5 grams to about 1.5 grams.

Aspects of the apparatuses and methods described herein can provide for preparation and administration of one or more substances, where the one or more substances, a mixing/compounding container, a measuring container, and a dispensing container are provided to a user in a unified manner. In this manner, deficiencies in existing techniques are overcome, which typically require that these components and/orcomponents performing these functions (e.g. a bottle of liquid, concentrated contrast media, a measuring device such as a syringe or other measuring device, and one or two cups for dispensing) be purchased separately and combined (e.g. opening the bottle of prepackage contrast agent, measuring the required amount of agent, and mixing/compounding in a separate cup) to create a unit dose. As a result, preparation time of a unit cost, and the cost associated therewith, is reduced with use of the apparatus and methods described herein, which in turn can provide time and cost savings for providers and patients alike.

While various embodiments of the invention have been described above, it should be understood that they have been presented by way of example only, and not limitation. Where methods and steps described above indicate certain events occurring in certain order, those of ordinary skill in the art having the benefit of this disclosure would recognize that the ordering of certain steps may be modified.

## Claims

1. An apparatus providing a concentrated contrast agent for oral consumption by a user, comprising:
a container body defining an opening in fluid communication with an interior of the container body and including a unit dose of a concentrated contrast agent within the interior of the container body such that the interior has a head space to receive a volume of liquid through the opening to dilute the concentrated contrast agent to a select dilution strength, wherein the unit dose of the concentrated contrast agent is configured to be diluted to the select dilution strength with a volume of the liquid receivable through the opening and within the interior of the container body;
a cap coupled to the container body and enclosing the opening; and
a barrier member defining an interior, wherein the container body and the cap are collectively disposed within the interior of the barrier member, and wherein the barrier member protects the concentrated contrast agent from light and/or moisture.

2. The apparatus of claim 1, wherein the concentrated contrast agent is an iodinated contrast agent.

3. The apparatus of claim 2, wherein the iodinated contrast agent is iohexol. 1

4. The apparatus of claim 1, wherein the concentrated contrast agent is free of functional excipients or additives.

5. The apparatus of claim 1, wherein the concentrated contrast agent is in a powder form.

6. The apparatus of claim 3, wherein the unit dose of iohexol is from about 0.5 gl to about 25 gl with the volume of the liquid receivable from about 80 mL to about 2,000 mL.

7. The apparatus of claim 3, wherein the unit dose of iohexol is from about 0.2 gI to about 75 gI with the volume of the liquid receivable from about 4 mL to about 250 mL.

8. The apparatus of claim 1, wherein the container body is configured to be used by a patient to orally consume the concentrated contrast agent through the opening of the container body when the concentrated contrast agent has been diluted with the volume of liquid.

9. The apparatus of claim 1, wherein the container body includes at least a portion that is transparent and a plurality of indications disposed on the portion that is transparent, each indication from the plurality of indications corresponding to a volume of liquid to be received within the interior of the container body such that a corresponding predetermined dilution of the unit dose of the concentrated contrast agent can be achieved.

10. The apparatus of claim 1, wherein the container body includes at least a portion that is transparent, the apparatus further comprising:
a label disposed on the container body and including a plurality of indications each corresponding to a volume of liquid to be received within the interior of the container body such that a corresponding predetermined dilution of the unit dose of the concentrated contrast agent can be achieved, the plurality of indications being disposed on the label adjacent the transparent portion.

11. The apparatus of claim 1, wherein the barrier member includes a laminate of polymer and aluminum.

12. The apparatus of claim 1, wherein the barrier member is sealed with one of an ultrasonic seal and a heat seal.

13. A method for providing a concentrated contrast agent for oral consumption by a user, comprising:
removing a container body with a cap disposed thereon from an interior of a barrier member, wherein the barrier member is configured to shield a unit dose of a concentrated contrast agent from light and/or moisture and wherein the container body has the unit dose of the concentrated contrast agent disposed within an interior of the container body such that the interior of the container body has a head space to receive a volume of liquid through the opening to dilute the concentrated contrast agent to a select dilution strength_{.};
removing the cap from the container body;
adding a volume of a liquid to the interior of the container body such that the concentrated contrast agent is diluted to a select dilution strength; and
providing the container body to a user.

14. The method of claim 13, further comprising:
after the adding the volume of liquid, coupling the cap to the container body; and
agitating the container body such that the volume of liquid is mixed with the concentrated contrast agent.

15. The method of claim 13, wherein the concentrated contrast agent is iohexol in a powder form.

## Patentansprüche

1. Vorrichtung, die ein konzentriertes Kontrastmittel zur oralen Einnahme durch einen Anwender bereitstellt, umfassend:
einen Behälterkörper, der eine Öffnung in Fluidverbindung mit einem Innenraum des Behälterkörpers definiert und eine Einheitsdosis eines konzentrierten Kontrastmittels im Innenraum des Behälterkörpers aufweist, so dass der Innenraum einen Kopfraum zur Aufnahme eines Flüssigkeitsvolumens durch die Öffnung zur Verdünnung des konzentrierten Kontrastmittels bis zu einem ausgewählten Verdünnungsgrad aufweist, wobei die Einheitsdosis des konzentrierten Kontrastmittels ausgelegt ist, mit einem Flüssigkeitsvolumen, das durch die Öffnung und in dem Innenraum des Behälterkörpers aufgenommen werden kann, bis zu dem ausgewählten Verdünnungsgrad verdünnt zu werden;
eine mit dem Behälterkörper verbundene und die Öffnung umschließende Kappe; und
ein Barriereelement, das einen Innenraum definiert, wobei der Behälterkörper und die Kappe gemeinsam in dem Innenraum des Barriereelements angeordnet sind, und wobei das Barriereelement das konzentrierte Kontrastmittel vor Licht und/oder Feuchtigkeit schützt.

2. Vorrichtung nach Anspruch 1, wobei das konzentrierte Kontrastmittel ein iodhaltiges Kontrastmittel ist.

3. Vorrichtung nach Anspruch 2, wobei das iodhaltige Kontrastmittel Iohexol ist.

4. Vorrichtung nach Anspruch 1, wobei das konzentrierte Kontrastmittel keine funktionalen Hilfsstoffe oder Zusatzstoffe enthält.

5. Vorrichtung nach Anspruch 1, wobei das konzentrierte Kontrastmittel in einer Pulverform vorliegt.

6. Vorrichtung nach Anspruch 3, wobei die Einheitsdosis von Iohexol ungefähr 0,5 gI bis ungefähr 25 gI bei einem aufnehmbaren Flüssigkeitsvolumen von ungefähr 80 ml bis ungefähr 2.000 ml beträgt.

7. Vorrichtung nach Anspruch 3, wobei die Einheitsdosis von Iohexol ungefähr 0,2 gI bis ungefähr 75 gI bei einem aufnehmbaren Flüssigkeitsvolumen von ungefähr 4 ml bis ungefähr 250 ml beträgt.

8. Vorrichtung nach Anspruch 1, wobei der Behälterkörper ausgelegt ist, von einem Patienten zur oralen Einnahme des konzentrierten Kontrastmittels durch die Öffnung des Behälterkörpers verwendet zu werden, nachdem das konzentrierte Kontrastmittel mit dem Flüssigkeitsvolumen verdünnt wurde.

9. Vorrichtung nach Anspruch 1, wobei der Behälterkörper zumindest einen transparenten Abschnitt und eine Vielzahl von Markierungen auf dem transparenten Abschnitt aufweist, wobei jede Markierung der Vielzahl von Markierungen einem Flüssigkeitsvolumen entspricht, das im Innenraum des Behälterkörpers aufgenommen werden soll, so dass eine entsprechende vorbestimmte Verdünnung der Einheitsdosis des konzentrierten Kontrastmittels erreicht werden kann.

10. Vorrichtung nach Anspruch 1, wobei der Behälterkörper zumindest einen transparenten Abschnitt aufweist, wobei die Vorrichtung ferner das Folgende umfasst:
ein Etikett, das auf dem Behälterkörper angeordnet ist und eine Vielzahl von Markierungen aufweist, die jeweils einem Flüssigkeitsvolumen entsprechen, das im Innenraum des Behälterkörpers aufgenommen werden soll, so dass eine entsprechende vorbestimmte Verdünnung der Einheitsdosis des konzentrierten Kontrastmittels erreicht werden kann, wobei die Vielzahl von Markierungen auf dem Etikett neben dem transparenten Abschnitt angeordnet ist.

11. Vorrichtung nach Anspruch 1, wobei das Barriereelement ein Laminat aus Polymer und Aluminium aufweist.

12. Vorrichtung nach Anspruch 1, wobei das Barriereelement mit einer Ultraschalldichtung oder mit einem Heißsiegel abgedichtet ist.

13. Verfahren zur Bereitstellung eines konzentrierten Kontrastmittels zur oralen Einnahme durch einen Anwender, umfassend:
Entfernen eines Behälterkörpers mit einer darauf angeordneten Kappe aus einem Innenraum eines Barriereelements, wobei das Barriereelement ausgelegt ist, eine Einheitsdosis eines konzentrierten Kontrastmittels vor Licht und/oder Feuchtigkeit abzuschirmen und wobei der Behälterkörper die Einheitsdosis des konzentrierten Kontrastmittels in einem Innenraum des Behälterkörpers aufweist, so dass der Innenraum des Behälterkörpers einen Kopfraum zur Aufnahme eines Flüssigkeitsvolumens durch die Öffnung zur Verdünnung des konzentrierten Kontrastmittels bis zu einem ausgewählten Verdünnungsgrad aufweist;
Entfernen der Kappe von dem Behälterkörper;
Hinzufügen eines Flüssigkeitsvolumens zum Innenraum des Behälterkörpers, so dass das konzentrierte Kontrastmittel bis zu einem ausgewählten Verdünnungsgrad verdünnt wird; und
Bereitstellen des Behälterkörpers für einen Anwender.

14. Verfahren nach Anspruch 13, ferner umfassend:
nach dem Hinzufügen des Flüssigkeitsvolumens, Verbinden der Kappe mit dem Behälterkörper; und
Schütteln des Behälterkörpers, so dass das Flüssigkeitsvolumen mit dem konzentrierten Kontrastmittel vermischt wird.

15. Verfahren nach Anspruch 13, wobei das konzentrierte Kontrastmittel Iohexol in einer Pulverform ist.

## Revendications

1. Appareil fournissant un agent de contraste concentré pour consommation orale par un utilisateur, comprenant :
un corps de récipient définissant une ouverture en communication fluidique avec un intérieur du corps de récipient et incluant une dose unitaire d'un agent de contraste concentré à l'intérieur du corps de récipient, de sorte que l'intérieur comporte un volume de tête pour recevoir un volume de liquide à travers l'ouverture destiné à diluer l'agent de contraste concentré jusqu'à une concentration choisie, où la dose unitaire de l'agent de contraste concentré est configurée de sorte à être diluée jusqu'à la concentration choisie avec un volume du liquide pouvant être reçu via l'ouverture et à l'intérieur du corps de récipient ;
un bouchon couplé au corps de récipient et fermant l'ouverture ; et
un élément barrière définissant un intérieur, où le corps de récipient et le bouchon sont disposés collectivement à l'intérieur de l'élément barrière, et
où l'élément barrière protège l'agent de contraste concentré de la lumière et/ou de l'humidité.

2. Appareil selon la revendication 1, où l'agent de contraste concentré est un agent de contraste iodé.

3. Appareil selon la revendication 2, où l'agent de contraste iodé est l'iohexol.

4. Appareil selon la revendication 1, où l'agent de contraste concentré est exempt d'excipients ou adjuvants fonctionnels.

5. Appareil selon la revendication 1, où l'agent de contraste concentré se présente sous forme de poudre.

6. Appareil selon la revendication 3, où la dose unitaire d'iohexol est comprise entre environ 0,5 gI et environ 25 gI avec le volume du liquide pouvant être reçu compris entre environ 80 mL et environ 2000 mL.

7. Appareil selon la revendication 3, où la dose unitaire d'iohexol est comprise entre environ 0,2 gI et environ 75 gI avec le volume du liquide pouvant être reçu compris entre environ 4 mL et environ 250 mL.

8. Appareil selon la revendication 1, où le corps de récipient est configuré pour être utilisé par un patient pour qu'il consomme par voie orale l'agent de contraste concentré via l'ouverture du corps de récipient lorsque l'agent de contraste concentré a été dilué par le volume de liquide.

9. Appareil selon la revendication 1, où le corps de récipient inclut au moins une portion qui est transparente et une multitude d'indications disposées sur la portion qui est transparente, chaque indication de la multitude d'indications correspondant à un volume de liquide devant être reçu à l'intérieur du corps de récipient de sorte qu'une dilution prédéterminée correspondante de la dose unitaire de l'agent de contraste concentré puisse être obtenue.

10. Appareil selon la revendication 1, où le corps de récipient inclut au moins une portion qui est transparente, l'appareil comprenant en outre :
une étiquette disposée sur le corps de récipient et incluant une multitude d'indications, chacune correspondant à un volume de liquide devant être reçu à l'intérieur du corps de récipient de sorte qu'une dilution prédéterminée correspondante de la dose unitaire de l'agent de contraste concentré puisse être obtenue, la multitude d'indications étant disposée sur l'étiquette adjacente à la portion transparente.

11. Appareil selon la revendication 1, où l'élément barrière inclut un laminé de polymère et d'aluminium.

12. Appareil selon la revendication 1, où l'élément barrière est scellé avec l'un des membres du groupe choisi parmi un scellage par ultrasons et un scellage thermique.

13. Procédé permettant de fournir un agent de contraste concentré pour consommation orale par un utilisateur, comprenant :
le retrait d'un corps de récipient doté d'un bouchon disposé sur celui-ci de l'intérieur d'un élément barrière, où l'élément barrière est configuré pour protéger une dose unitaire d'un agent de contraste concentré de la lumière et/ou de l'humidité et où le corps de récipient présente la dose unitaire de l'agent de contraste concentré disposée à l'intérieur du corps de récipient, de sorte que l'intérieur du corps de récipient présente un volume de tête destiné à recevoir un volume de liquide à travers l'ouverture pour diluer l'agent de contraste concentré jusqu'à une concentration choisie ;
le retrait du bouchon du corps de récipient ;
l'ajout d'un volume de liquide à l'intérieur du corps de récipient de sorte que l'agent de contraste concentré soit dilué jusqu'à une concentration choisie ; et
le fait de fournir le corps de récipient à un utilisateur.

14. Procédé selon la revendication 13, comprenant en outre :
après l'ajout du volume de liquide, le couplage du bouchon au corps de récipient ; et
l'agitation du corps de récipient de sorte que le volume de liquide soit mélangé à l'agent de contraste concentré.

15. Procédé selon la revendication 13, où l'agent de contraste concentré est de l'iohexol sous forme de poudre.
